(19) Europäisches Patentamt
European Patent Office
Office européen des brevets

(11) Numéro de publication: **0 606 175 B1**

# (12) FASCICULE DE BREVET EUROPEEN

(45) Date de publication du fascicule du brevet: **11.10.95**

(51) Int. Cl.[6]: **C07D 213/75**, A61K 31/44

(21) Numéro de dépôt: **94400009.0**

(22) Date de dépôt: **04.01.94**

Le dossier contient des informations techniques présentées postérieurement au dépôt de la demande et ne figurant pas dans le présent fascicule.

## (54) Nouveaux dérivés 2-cyano 3-hydroxy propénamides, leur procédé de préparation, leur application comme médicaments et les compositions pharmaceutiques les renfermant.

(30) Priorité: **05.01.93 GB 9300083**

(43) Date de publication de la demande:
**13.07.94 Bulletin 94/28**

(45) Mention de la délivrance du brevet:
**11.10.95 Bulletin 95/41**

(84) Etats contractants désignés:
**AT BE CH DE DK ES FR GB GR IE IT LI LU NL PT SE**

(56) Documents cités:
**EP-A- 0 372 470** **EP-A- 0 484 223**
**EP-A- 0 533 573** **WO-A-91/17748**
**DE-A- 2 555 789** **US-A- 4 435 407**

**JOURNAL OF THE CHEMICAL SOCIETY, PERKIN TRANSACTIONS 1 vol. 17 , 1992 , LETCHWORTH, GB; pages: 2203-2213; C.A. AXTON ET AL.: "Novel immunosuppressive butenamides"**

(73) Titulaire: **ROUSSEL UCLAF**
**102 Route de Noisy**
**F-93230 Romainville (FR)**

(72) Inventeur: **Kuo, Elizabeth Anne**
**8 Bullfinch Close,**
**Covingham**
**Swindon,**
**Wiltshire SN3 5PH (GB)**

(74) Mandataire: **Vieillefosse, Jean-Claude et al**
**ROUSSEL UCLAF**
**Département des Brevets**
**102, Route de Noisy**
**F-93235 Romainville (FR)**

**EP 0 606 175 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux 2-cyano-3-hydroxy-propenamides, leurs formes tautomères et leurs sels, ainsi que le procédé de préparation, l'application à titre de médicaments de ces nouveaux produits et les compositions les renfermant. Des dérivés du 2-cyano 3-hydroxy propénamide sont décrits dans EP-A 484223 et dans WO-A 91-17748.

L'invention a pour objet de nouveaux 2-cyano-3-hydroxy-propenamides répondant à la formule générale (I) :

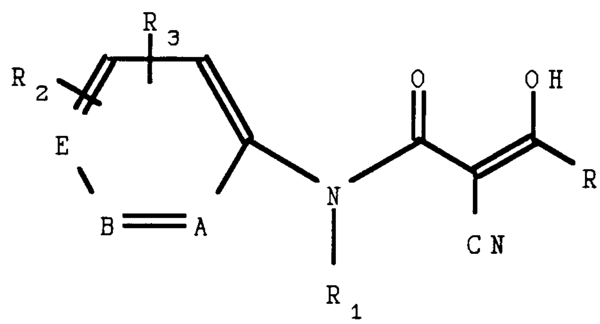

(I)

dans laquelle :

- A, B et E représentent chacun un groupement =CH- ou un atome d'azote, étant entendu que l'un au moins de A, B, ou E représente un atome d'azote,
- R représente un radical cycloalkyle renfermant de 3 à 6 atomes de carbone, un radical alkenyle renfermant de 2 à 6 atomes de carbone, un radical alkynyle renfermant de 2 à 6 atomes de carbone,
- $R_1$ représente un atome d'hydrogène, un radical alkyle renfermant de 1 à 3 atomes de carbone,
- $R_2$ et $R_3$, identiques ou différents, représentent un atome d'hydrogène, un atome d'halogène, un groupement cyano, un groupement nitro, un radical alkyle, linéaire ou ramifié, renfermant de 1 à 6 atomes de carbone, un radical cycloalkyle renfermant de 3 à 6 atomes de carbone, un radical alcoxy, linéaire ou ramifié, renfermant de 1 à 6 atomes de carbone, un radical alkylthio renfermant de 1 à 6 atomes de carbone, un radical $-(CH_2)_m-CX_3$ , un radical $-O-(CH_2)_m-CX_3$ , un radical $-S-(CH_2)_m-CX_3$, un radical $-O-(CX_2)_m-CX_3$, un radical $-S-(CX_2)_m-CX_3$, radicaux dans lesquels m représente un nombre entier compris entre 0 et 3 et X représente un atome d'halogène, ou un groupement $-CO-R_4$ dans lequel $R_4$ représente un atome d'hydrogène, un radical alkyle, linéaire ou ramifié, renfermant de 1 à 6 atomes de carbone, un radical cycloalkyle renfermant de 3 à 6 atomes de carbone,

ou $R_2$ et $R_3$ , forment ensemble un groupement $-O-CH_2-O-$ , leurs formes tautomères, ainsi que leurs sels d'addition avec les bases minérales ou organiques.

Dans la formule générale (I) et dans ce qui suit :

- par radical cycloalkyle renfermant de 3 à 6 atomes de carbone, on entend un radical cyclopropyle, cyclobutyle, cyclopentyle ou cyclohexyle;
- par radical alkenyle renfermant de 2 à 6 atomes de carbone, on entend de préférence des radicaux de formule :

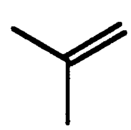 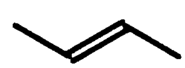 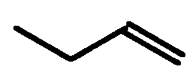

- par radical alkynyle renfermant de 2 à 6 atomes de carbone, on entend de préférence un radical de formule :

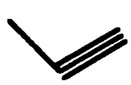

- par radical alkyle renfermant de 1 à 3 atomes de carbone, on entend un radical méthyle, éthyle, propyle, isopropyle;
- par atome d'halogène, on entend un atome de fluor, de chlore, de brome ou d'iode et de préférence un atome de fluor, de chlore ou de brome;
- par radical alkyle renfermant de 1 à 6 atomes de carbone, on entend de préférence un radical méthyle, éthyle, propyle, isopropyle, butyle linéaire ou ramifié, pentyle linéaire ou ramifié, hexyle linéaire ou ramifié,
- par radical alcoxy comportant de 1 à 6 atomes de carbone, on entend par exemple, un radical méthoxy, éthoxy, propoxy, isopropoxy, butoxy linéaire ou ramifié, pentyloxy linéaire ou ramifié, hexyloxy linéaire ou ramifié,
- par radical alkylthio comportant de 1 à 6 atomes de carbone, on entend par exemple, un radical méthylthio, éthylthio, propylthio, isopropylthio, butylthio linéaire ou ramifié, pentylthio linéaire ou ramifié, hexylthio linéaire ou ramifié,
- par radicaux $-(CH_2)_m-CX_3$, $-O-(CH_2)_m-CX_3$, $-S-(CH_2)_m-CX_3$, $-O-(CX_2)_m-CX_3$ et $-S-(CX_2)_m-CX_3$ dans lesquels m représente un nombre entier compris entre 0 et 3, on entend par exemple des radicaux : $-CF_3$ , $-CH_2-CF_3$ , $-(CH_2)_2-CF_3$ , $-(CH_2)_3-CF_3$ , $-O-CF_3$ , $-O-CH_2-CF_3$ , $-O-(CH_2)_2-CF_3$ , $-O-(CH_2)_3-CF_3$ , $-S-CF_3$ , $-S-CH_2-CF_3$ , $-S-(CH_2)_2-CF_3$ , $-S-(CH_2)_3-CF_3$ , $-O-CF_2-CF_3$, $-S -CF_2-CF_3$.

Le groupement de formule :

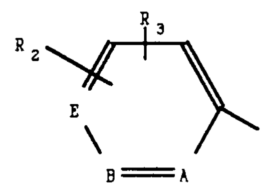

peut notamment représenter un groupement : N-(2-chloropyrid-5-yl)- , N-(4-methyl-5-nitropyrid-2-yl)- , N-(5-trifluoromethylpyrid-2-yl)-, N-(5-chloropyrid-2-yl)- , N-(5-bromopyrid-2-yl)- , N-(5-nitropyrid-2-yl)-N-(pyrid-4-yl)-ou N-(3,5--dichloropyrid-2-yl)-.

Les sels d'addition avec les bases minérales ou organiques peuvent être, par exemple, les sels formés avec les bases minérales tels que les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris(hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

Parmi les produits, objet de l'invention, on peut citer notamment les dérivés répondant à la formule (I) ci-dessus, ainsi que leurs sels, caractérisés en ce que dans ladite formule (I), R représente un radical cyclopropyle ou des radicaux de formule :

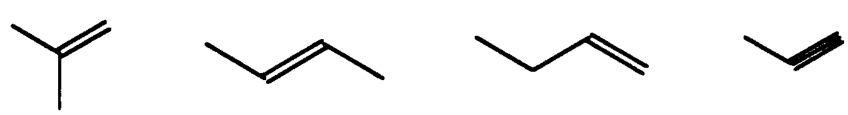

A, B, E, $R_1$, $R_2$ et $R_3$ ayant la signification indiquée ci-dessus ainsi que leurs sels d'addition avec les bases minérales ou organiques.

Parmi ces derniers, on peut citer plus particulièrement les dérivés répondant à la formule (I) ci-dessus, caractérisés en ce que dans ladite formule (I),
$R_1$ représente un atome d'hydrogène ou un radical méthyle, A, B, E, R, $R_2$ et $R_3$ ont la signification déjà indiquée, ainsi que leurs sels d'addition avec les bases minérales ou organiques.

Parmi ces derniers on retient plus particulièrement les dérivés de formule (I) dans laquelle :
$R_1$ représente un atome d'hydrogène ou un radical méthyle, $R_2$ et $R_3$ , identiques ou différents, représentent un atome d'hydrogène, un atome de chlore ou de brome, un groupement cyano, un groupement nitro, un radical méthyle, un radical cyclopropyle, un radical méthoxy, un radical méthylthio, un

radical $-(CH_2)_m-CF_3$, un radical $-O-(CH_2)_m-CF_3$, un radical $-S-(CH_2)_m-CF_3$, $-O-(CF_2)_m-CF_3$, $-S-(CF_2)_m-CF_3$, radicaux dans lesquels m représente un nombre entier compris entre 0 et 3, ou un groupement $-CO-R_4$ dans lequel $R_4$ représente un atome d'hydrogène, un radical méthyle, un radical cyclopropyle, ou $R_2$ et $R_3$ , forment ensemble un groupement $-O-CH_2-O-$,
A, B, E et R ont la signification déjà indiquée, leurs formes tautomères, ainsi que leurs sels d'addition avec les bases minérales ou organiques et tout particulèrement les derivés de formule (I) dans laquelle R représente un radical cyclopropyle, $R_1$ représente un atome d'hydrogène ou un radical méthyle, $R_2$ et $R_3$, identiques ou différents, représentent un atome d'hydrogène, un atome de chlore ou de brome, un groupement nitro, un radical méthyle, un radical trifluorométhyle, A, B, et E ont la signification déjà indiquée, leurs formes tautomères, ainsi que leurs sels d'addition avec les bases minérales ou organiques.

Parmi ces derniers, on retient tout particulièrement les dérivés de formule (I) dont les noms suivent :

- N-(2-chloropyrid-5-yl) 2-cyano-3-cyclopropyl-3-hydroxy-2-propenamide,
- 2-cyano-3-cyclopropyl-3-hydroxy-N-(4-methyl-5-nitropyrid-2-yl)-2-propenamide,
- 2-cyano-3-cyclopropyl-3-hydroxy-N-(5-trifluoromethylpyrid-2-yl)-2-propenamide,
- N-(5-chloropyrid-2-yl) 2-cyano-3-cyclopropyl-3-hydroxy-2-propenamide,
- N-(5-bromopyrid-2-yl) 2-cyano-3-cyclopropyl-3-hydroxy-2-propenamide,
- 2-cyano-3-cyclopropyl-3-hydroxy-N-(5-nitropyrid-2-yl)-2-propenamide,
- 2-cyano-3-cyclopropyl-3-hydroxy-N-(pyrid-4-yl)-2-propenamide,
- 2-cyano-3-cyclopropyl-3-hydroxy-N-(3,5-dichloropyrid-2-yl)-2-propenamide,

ainsi que leurs sels d'addition avec les bases minérales ou organiques.

L'invention a également pour objet un procédé de préparation des nouveaux 2-cyano-3-hydroxy-propenamides tels que définis par la formule (I) ci-dessus, ainsi que de leurs sels, caractérisé en ce que l'on fait réagir un produit de formule (II) :

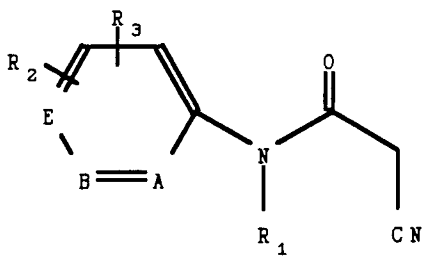

(II)

dans laquelle A, B, E, $R_1$, $R_2$ et $R_3$ ont la signification déjà indiquée, avec successivement de l'hydrure de sodium, si nécessaire en présence d'un catalyseur, puis avec le dérivé fonctionnel d'un acide de formule (III):

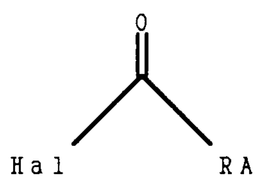

(III)

dans laquelle Hal représente un atome d'halogène et $R_A$ a la signification de R déjà indiquée, ou $R_A$ représente un radical R dûment protégé, pour obtenir un produit de formule ($I_A$):

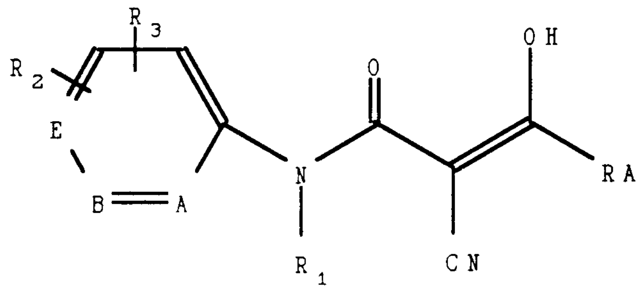

$(I_A)$

dans laquelle A, B, E, $R_A$, $R_1$, $R_2$ et $R_3$ ont la signification déjà indiquée, puis, le cas échéant, déprotège le produit de formule ($I_A$) ainsi obtenu dans lequel $R_A$ représente un radical R dûment protégé, pour obtenir un produit de formule (I) correspondant, que l'on isole et si désiré salifie.

Dans des conditions préférentielles de mise en oeuvre de l'invention, le procédé de préparation ci-dessus décrit est caractérisé en ce que :

- la réaction du produit de formule (II) avec l'hydrure de sodium est effectuée au sein d'un solvant organique anhydre tel que le tétrahydrofuranne ou le dichloromethane en présence si nécessaire d'un catalyseur capable de solvater l'hydrure de sodium, tel que l'imidazole,
- la réaction avec le dérivé de formule (III) est effectuée de préférence au sein d'un solvant organique tel que le tétrahydrofuranne ou le dichlorométhane,
- la réaction du dérivé de formule (III) peut être effectuée à basse température, par exemple entre -80 ° C et -50 ° C ou aux environs de 0 ° C ou encore à température ambiante vers 25 ° C,
- le dérivé de formule (III) est de préférence un chlorure d'acide ou un fluorure d'acide. On peut citer par exemple le fluorure de propynoyle qui peut être préparé par action de l'acide propiolique sur du fluorure de benzoyle suivi d'une distillation du milieu réactionnel,
- le radical R peut être protégé par un groupement arylseleno tel qu'un groupement phenylseleno,
- la déprotection peut être effectuée par oxydation au moyen d'un peroxyde tel que le peroxyde d'hydrogène en présence ou non d'un mélange de solvants organiques tel que le mélange methanol-dichloromethane.

L'invention a également pour objet une variante du procédé de préparation des nouveaux 2-cyano-3-hydroxy-propenamides tels que définis par la formule (I) ci-dessus, dans laquelle A, B, E, $R_1$, $R_2$ et $R_3$ ont la signification déjà indiquée, et R représente un radical cycloalkyle renfermant de 3 à 6 atomes de carbone, ainsi que de leurs sels, caractérisé en ce que l'on fait réagir un produit de formule (IV) :

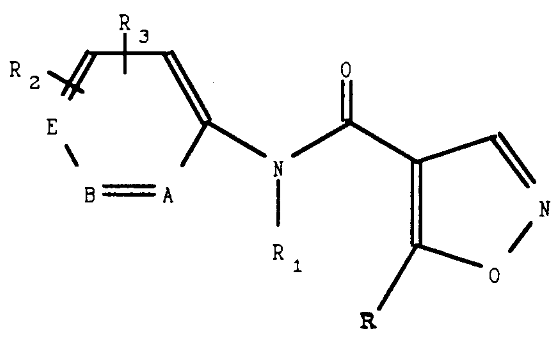

(IV)

dans laquelle A, B, E, R, $R_1$, $R_2$ et $R_3$ ont la signification déjà indiquée, avec une base forte pour obtenir le produit de formule (I) recherché, que l'on isole et si désiré salifie.

Dans des conditions préférentielles de mise en oeuvre de de l'invention, cette variante du procédé de préparation ci-dessus décrite est caractérisé en ce que la réaction du produit de formule (IV) avec la base forte est effectuée au reflux du mélange réactionnel.

Les produits de formule (I) présentent un caractère acide. On peut avantageusement préparer les sels d'addition des produits de formule (I) en faisant réagir, en proportions sensiblement stoéchiométriques, une base minérale ou organique avec lesdits produits de formule (I). Les sels peuvent être préparés sans isoler les acides correspondants.

Les produits, objet de la présente invention, possèdent de très intéressantes propriétés pharmacologiques. On note en particulier une remarquable activité anti-inflammatoire. Ils inhibent d'une part les

phénomènes inflammatoires provoqués par des agents irritants, et d'autre part les réactions d'hypersensibilité retardée, en empêchant l'activation des cellules immunitaires par un antigène spécifique.

Ces propriétés sont illustrées plus loin dans la partie expérimentale.

Ces propriétés justifient l'utilisation des nouveaux 2-cyano-3-hydroxy-propenamides répondant à la formule (I), ainsi que de leurs sels d'addition avec les bases pharmaceutiquement acceptables, à titre de médicaments.

La présente invention a aussi également pour objet l'application à titre de médicaments des nouveaux 2-cyano-3-hydroxy-propenamides tels que définis par la formule générale (I), ainsi que de leurs sels d'addition avec les bases minérales ou organiques pharmaceutiquement acceptables.

Parmi les médicaments, objet de l'invention, on retient notamment les médicaments, caractérisés en ce qu'ils sont constitués par les nouveaux 2-cyano-3-hydroxy-propenamides répondant à la formule (I) dans laquelle R représente un radical cyclopropyle ou des radicaux de formule :

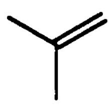 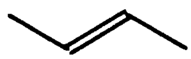 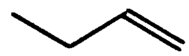 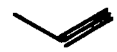

A, B, E, $R_1$, $R_2$ et $R_3$ ayant la signification déjà indiquée, ainsi que leurs sels d'addition avec les bases minérales ou organiques pharmaceutiquement acceptables.

Parmi les médicaments, objet de l'invention, on retient plus particulièrement ceux répondant à la formule (I) ci-dessus dans laquelle $R_1$ représente un atome d'hydrogène ou un radical méthyle,
A, B, E, R, $R_2$ et $R_3$ ont la signification déjà indiquée, ainsi que leurs sels d'addition avec les bases minérales ou organiques pharmaceutiquement acceptables.

Parmi les médicaments préférés de l'invention, on retient tout particulièrement les produits de formule (I) dans laquelle $R_1$ représente un atome d'hydrogène ou un radical méthyle, $R_2$ et $R_3$, identiques ou différents, représentent un atome d'hydrogène, un atome de chlore ou de brome, un groupement -CN, un groupement $-NO_2$, un radical méthyle, un radical cyclopropyle, un radical méthoxy, un radical méthylthio, un radical $-(CH_2)_m-CF_3$, un radical $-O-(CH_2)_m-CF_3$, un radical $-S-(CH_2)_m-CF_3$, $-O-(CF_2)_m-CF_3$, $-S-(CF_2)_m-CF_3$, radicaux dans lesquels m représente un nombre entier compris entre 0 et 3, ou un groupement $-CO-R_4$ dans lequel $R_4$ représente un atome d'hydrogène, un radical méthyle, un radical cyclopropyle, ou $R_2$ et $R_3$, forment ensemble un groupement $-O-CH_2-O-$, A, B, E et R ont la signification déjà indiquée, leurs formes tautomères ainsi que leurs sels d'addition avec les bases minérales ou organiques pharmaceutiquement acceptables, et notamment les dérivés de formule (I) dont les noms suivent:

- N-(2-chloropyrid-5-yl) 2-cyano-3-cyclopropyl-3-hydroxy-2-propenamide,
- 2-cyano-3-cyclopropyl-3-hydroxy-N-(4-methyl-5-nitropyrid-2-yl)-2-propenamide,
- 2-cyano-3-cyclopropyl-3-hydroxy-N-(5-trifluoromethylpyrid-2-yl)-2-propenamide,
- N-(5-chloropyrid-2-yl) 2-cyano-3-cyclopropyl-3-hydroxy-2-propenamide,
- N-(5-bromopyrid-2-yl) 2-cyano-3-cyclopropyl-3-hydroxy-2-propenamide,
- 2-cyano-3-cyclopropyl-3-hydroxy-N-(5-nitropyrid-2-yl)-2-propenamide,
- 2-cyano-3-cyclopropyl-3-hydroxy-N-(pyrid-4-yl)-2-propenamide,
- 2-cyano 3-cyclopropyl-3-hydroxy N-(3,5-dichloropyrid-2-yl) 2-propenamide,

ainsi que leurs sels d'addition avec les bases minérales ou organiques pharmaceutiquement acceptables.

Ces médicaments trouvent, par exemple, leur emploi dans le traitement de l'arthrite rhumatoïde et des maladies inflammatoires chroniques d'origine immune ou non (greffes, transplantations d'organes, etc...).

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les bases pharmaceutiquement acceptables à titre de principe actif.

A titre de médicaments, les dérivés répondant à la formule (I) et leurs sels d'addition avec les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être

incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) peuvent être préparés par réaction d'un produit de formule (V) :

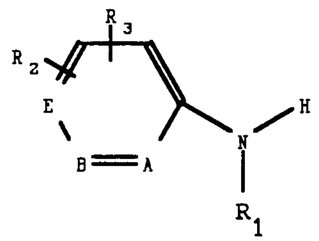

(V)

dans laquelle A, B, E, $R_1$, $R_2$ et $R_3$ ont la signification déjà indiquée, avec de l'acide cyanoacétique en présence de dicyclohexylcarbodiimide ou de pentachlorure de phosphore au sein d'un solvant organique anhydre tel que le tetrahydrofuranne ou le dichloromethane. La réaction en présence de dicyclohexylcarbodiimide au sein de tetrahydrofuranne anhydre est appelée méthode A dans ce qui suit. La réaction en présence de pentachlorure de phosphore au sein de dichloromethane anhydre est appelée méthode B dans ce qui suit.

Les produits de formule (IV) peuvent être préparés par réaction d'un produit de formule (V) tel que défini ci-dessus avec un chlorure d'acide de formule (VI) :

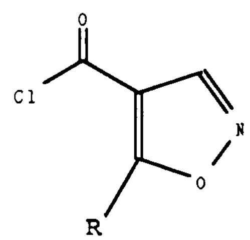

(VI)

dans lequel R a la signification déjà indiquée selon un procédé analogue au procédé décrit dans la demande de brevet WO91/17748.

Le chlorure d'acide de formule (VI) peut être préparé au départ de l'acide correspondant. L'acide peut lui-même être préparé selon des procédés décrits dans la littérature; on peut notamment citer la demande de brevet européen EP 326.107.

Il va être donné maintenant à titre non limitatif, des exemples de mise en oeuvre de l'invention.

## PREPARATION DES PRODUITS DE DEPART

**N-(2-chloropyrid-5-yl)-2-cyanoacetamide. (produit de départ de l'exemple 1)**

On ajoute à 0°C sous agitation 5,95g d'acide cyanoacétique et 14,44g de dicyclohexylcarbodiimide à 9g de 5-amino-2-chloropyridine en solution dans 150 cm3 de tétrahydrofuranne. Quand la réaction est complète, on filtre, évapore le solvant, reprend le résidu dans le chlorure de méthylène, filtre et sèche sous pression réduite. On obtient 10,56g de N-(2-chloropyrid-5-yl)-2-cyanoacetamide.

En opérant comme indiqué ci-dessus avec les modifications notées ci-après, on a préparé les produits suivants :

**N-(4-methyl-5-nitropyrid-2-yl)-2-cyanoacétamide. (produit de départ de l'exemple 2)**

On purifie le produit par chromatographie sur silice (éluant : hexane/acétate d'éthyle 60-40). On obtient le N-(4-methyl-5-nitropyrid-2-yl)-2-cyanoacétamide avec un rendement de 51%.

**N-(5-trifluorométhyl-pyrid-2-yl)-2-cyanoacétamide. (produit de départ de l'exemple 3)**

En utilisant 1,2 équivalents d'acide cyanoacétique et 1,2 équivalents de dicyclohexyl carbodiimide on obtient le N-(5-trifluoromethyl-pyrid-2-yl)-2-cyanoacétamide avec un rendement de 79%.

**N-(5-chloro-pyrid-2-yl)-2-cyanoacétamide. (produit de départ de l'exemple 4 )**

On opère avec 1 équivalent d'acide cyanoacétique et 1 équivalent de dicyclohexyl carbodiimide dans le chlorure de méthylène au reflux. Après évaporation du solvant, on reprend le résidu dans l'acétate d'éthyle et obtient le N-(5-chloro-pyrid-2-yl)-2-cyanoacétamide avec un rendement de 90%.

**N-(5-bromo-pyrid-2-yl)-2-cyanoacétamide. (produit de départ de l'exemple 5)**

On mélange 0,22g d'acide cyanoacétique à 0,54g de pentachlorure de phosphore en solution dans 8cm3 de chlorure de méthylène pendant 1 minute puis chauffe 30 minutes au reflux la solution obtenue et effectue un balayage d'azote. On ajoute 0,30g de 2-amino-5-bromopyridine et poursuit le reflux. Quand la réaction est terminée, on verse dans 4cm3 d'eau, agite pendant 30 minutes, filtre, sèche sous pression réduite et obtient 0,27g de N-(5-bromo-pyrid-2-yl)-2-cyano-acétamide.

En opérant comme indiqué ci-dessus, on a préparé les produits suivants :

**N-(5-nitro-pyrid-2-yl)-2-cyanoacétamide .(produit de départ de l'exemple 6).**

En utilisant au départ le 2-amino-5-nitro pyridine, on obtient le N-(5-nitro-pyrid-2-yl)-2-cyanoacétamide, avec un rendement de 42%.

**N-(3,5-dichloro-pyrid-2-yl)-2-cyanoacétamide . (produit de départ de l'exemple 8).**

En utilisant au départ le 2-amino-3,5-dichloro-pyridine, on obtient le N-(3,5-dichloro-pyrid-2-yl)-2-cyanoacétamide, avec un rendement de 29%.

**<u>EXEMPLE 1</u> : 2-cyano 3-cyclopropyl-3-hydroxy-N-(2-chloropyrid-5-yl) 2-propenamide.**

On ajoute à 0°C sous agitation 3,21g d'hydrure de sodium (à 80% dans l'huile) à 7g de N-(2-chloropyrid-5-yl)-2-cyano-acetamidedans 200cm3 de tétrahydrofuranne. On agite pendant 1 heure puis ajoute 48,6cm3 de chlorure cyclopropane carbonyle. Quand la réaction est terminée, on ajoute 1,251 d'eau, acidifie à pH 1 par addition d'acide chlorhydrique à 35% et maintient sous agitation pendant 30 minutes. On filtre le précipité obtenu et le lave à l'eau. On le triture dans l'acétate d'ethyle, le filtre, le sèche sous pression réduite et obtient 7,91g de produit attendu.

En opérant comme indiqué ci-dessus avec les modifications notées ci-après, on a préparé les produits suivants :

**<u>EXEMPLE 2</u> : 2-cyano 3-cyclopropyl-3-hydroxy-N-(4-méthyl-5-nityropyrid-2-yl) 2-propenamide.**

Après cristallisation dans le mélange acétate d'ethyle/hexane, on obtient le produit attendu avec un rendement de 87%.

**<u>EXEMPLE 3</u> : 2-cyano 3-cyclopropyl-3-hydroxy-N-(5-trifluorométhylpyrid-2-yl) 2-propenamide.**

Après cristallisation dans le mélange acétate d'ethyle/ether de pétrole (eb:40°-60°), on obtient le produit attendu avec un rendement de 86%.

**<u>EXEMPLE 4</u> : 2-cyano 3-cyclopropyl-3-hydroxy-N-(5-chloropyrid-2-yl) 2-propenamide.**

On utilise au départ de la réaction 2,4 équivalents d'hydrure de sodium et 1,2 équivalents de chlorure cyclopropane carbonyle. Après cristallisation dans 1' acétate d'ethyle, on obtient le produit attendu avec un rendement de 91%.

**EXEMPLE 5 : 2-cyano 3-cyclopropyl-3-hydroxy-N-(5-bromopyrid-2-yl) 2-propenamide.**

On utilise au départ de la réaction 2,4 équivalents d'hydrure de sodium, une quantité catalytique d'imidazole et 1,2 équivalents de chlorure cyclopropane carbonyle en opérant à 25°C. Après trituration dans 1' éther, filtration et séchage sous pression réduite, on obtient le produit attendu avec un rendement de 83%.

**EXEMPLE 6 : 2-cyano 3-cyclopropyl-3-hydroxy-N-(5-nitropyrid-2-yl) 2-propenamide.**

On utilise au départ de la réaction 2,4 équivalents d'hydrure de sodium, une quantité catalytique d'imidazole et 1,2 équivalents de chlorure cyclopropane carbonyle en opérant à 25°C. Après trituration dans 1' éther, filtration et séchage sous pression réduite, on obtient le produit attendu avec un rendement de 89%.

**EXEMPLE 7 : 2-cyano 3-cyclopropyl-3-hydroxy-N-(pyrid-4-yl) 2-propenamide.**

On chauffe 90 minutes au reflux 1,5g d'acide 5-cyclopropyl isoxazol-4-carboxylique (préparé comme indiqué dans le brevet européen N° 326 107 A1) et 20 cm3 de chlorure de thionyle. On évapore le milieu réactionnel sous pression réduite avec entrainement au toluène. On dissout le chlorure d'acide résultant dans 10 cm3 de chlorure de méthylène et ajoute 1g de 4-amino pyridine en suspension dans 50cm3 de chlorure de méthylène. On ajoute 0,77g de pyridine puis agite à température ambiante pendant 90 minutes. On filtre le produit obtenu, le dissout dans 100 cm3 de méthanol et ajoute 2cm3 de triethylamine. On chauffe 1 heure au reflux le milieu réactionnel, le verse dans l'eau puis l'acidifie à pH 1 par addition d'acide chlorhydrique concentré. On abandonne à 4°C pendant 16 heures, lave à l'eau le produit cristallisé obtenu, le sèche sous pression réduite et obtient 0,67g de produit attendu (Rendt : 45%).

**EXEMPLE 8 : 2-cyano 3-cyclopropyl-3-hydroxy-N-(3,5-dichloropyrid-2-yl) 2-propenamide.**

En opérant comme indiqué à l'exemple 1, on a préparé le produit attendu avec un rendement de 69%.

Les données spectrales, les rendements, les points de fusion, et les données analytiques pour les exemples 1 à 8 sont présentées dans le tableau I.

**Exemple 9 :**

Des comprimés ont été préparés selon la formulation suivante:

| | |
|---|---|
| Composé de l'exemple 1<br>Excipient pour un comprimé qsp | 20 mg<br>150 mg |
| (précisions sur l'excipient: lactose, amidon, talc, stéarate de magnésium). | |

**Exemple 10 :**

Des comprimés ont été préparés selon la formulation suivante:

| | |
|---|---|
| Composé de l'exemple 2<br>Excipient pour un comprimé qsp | 20 mg<br>150 mg |
| (précisions sur l'excipient: lactose, amidon, talc, stéarate de magnésium). | |

TABLEAU I

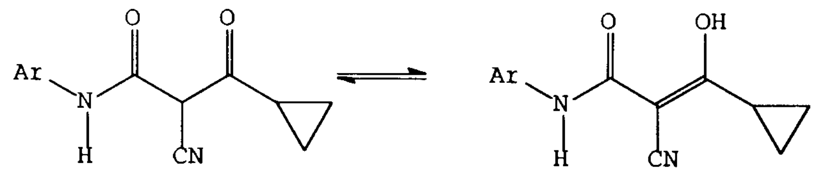

| Ex | Ar | Methode | F°C | IR $cm^{-1}$ | $^1H$ RMN δ | Formule Pds. Mol. | Analyse % Calc C | H | N | X |
|---|---|---|---|---|---|---|---|---|---|---|
| 1 | 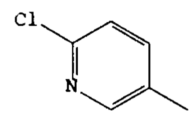 | A + C | 196-198 | 3280, 2195, 1560, 1515, 1455, 1275, 1110, 975, 885. | $CDCl_3$ 15.59[s,1H,O-H]; 8.51[d,J=2.5Hz,1H, Ar-H]; 8.00[s,dd,J=8.5Hz, 2.5Hz,2H,N-H,Ar-H];7.33[d,J=8.5Hz, 1H,Ar-H]; 2.14[m,1H, cyclopropyl-H]; 1.29[m,4H,cyclopropyl-H]. | $C_{12}H_{10}ClN_3O_2$ 263.69 | 54.66 | 3.82 | 15.94 | 13.45 |
| 2 | 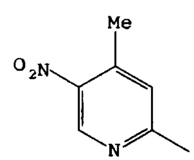 | A + C | 178 | (3400-2000)br, 3250, 2210, 1630, 1495, 1330, 1095, 985, 765. | DMSO-$d_6$ 13.14[brs,1H,O-H]; 11.86[vbrs,1H,N-H]; 8.94[s,1H,Ar-H]; 8.22[s,1H, Ar-H];2.57 [m,3H,Ar-Me]; 2.22[m,1H, cyclopropyl-H]; 0.79[m,4H, cyclopropyl-H]. | $C_{13}H_{12}N_4O_4$ 288.26 | 54.16 | 4.20 | 19.44 | |
| 3 | 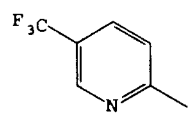 | A + C | 202-203 (decom-position) | 3395, 2205, 1635, 1580, 1520, 1395, 1325, 1125, 1075, 895. | $CDCl_3$ 15.38[brs,1H,O-H]; 8.60[s,1H,Ar-H]; 8.38[brs,1H,N-H]; 8.22[d,J=8.5Hz,1H, Ar-H]; 7.95[d,J=8.5Hz,1H, Ar-H];2.18[m,1H, cyclopropyl-H]; 1.29[m,4H, cyclopropyl-H]. | $C_{13}H_{10}F_3N_3O_2$ 297.23 | 52.53 | 3.39 | 14.14 | 19.18 |

TABLEAU I (suite)

| Ex | Ar | Methode | F°C | IR $cm^{-1}$ | $^1H$ RMN δ | Formule Pds. Mol. | Analyse % Calc | | | |
|---|---|---|---|---|---|---|---|---|---|---|
| | | | | | | | C | H | N | X |
| 4 | 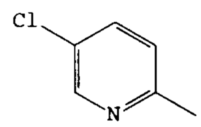 | A + C | 223-225 | (3600-2800)br, 3385, 2180, 1590, 1555, 1505, 1405, 1370, 1280, 1000, 760. | DMSO-$d_6$ 12.59[s,1H,N-H]; 8.24[m,2H,Ar-H]; 7.77[dd,J=9.0Hz, 2.5Hz,1H,Ar-H]; 2.20[m,1H,cyclopropyl-H]; 0.71[m,4H,cyclopropyl-H]. | $C_{12}H_{10}ClN_3O_2$ 263.69 | 54.66 54.42 | 3.82 3.91 | 15.94 15.69 | 13.45 |
| 5 | 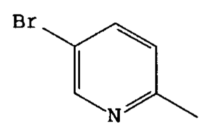 | B + C | 202-203 | (3300-1800)br, 3110, 2200, (1650-1530)br, 1145, 1005, 980, 770, 720. | $CDCl_3$ 13.00[brs,1H,N-H]; 8.36[d,J=2Hz,1H,Ar-H]; 8.07[m,2H,Ar-H]; 2.21[m,1H,cyclopropyl-H]; 0.79[m,4H,cyclopropyl-H]. | $C_{12}H_{10}BrN_3O_2$ 308.14 | 46.78 46.63 | 3.27 3.33 | 13.64 13.19 | 25.93 25.71 |
| 6 | 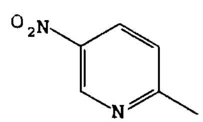 | B + C | 163-165 | (3400-1850)br, 3360, 2195, 1630, 1600, 1545, 1490, 1340, 1300, 980, 885, 630. | DMSO-$d_6$ 13.17[s,1H,N-H]; 9.07[d,J=3.0Hz,1H,ArH]; 8.50[dd,J=9.0Hz,1H,Ar-H]; 8.38[d,J=9.0Hz,1H,Ar-H]; 2.22[m,1H,cyclopropyl-H]; 0.77[m,4H,cyclopropyl-H]. | $C_{12}H_{10}N_4O_4$ 274.24 | 52.56 52.21 | 3.68 3.70 | 20.43 20.35 | |
| 7 | 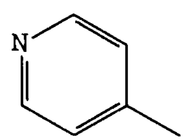 | D | >300 | (3600-2200)br, 3430, 2180, 1630, 1225, 1185, 1110, 985, 815. | DMSO-$d_6$ 14.17[brs,1H,O-H]; 13.51[s,1H,N-H]; 8.53[d,J=7.0Hz,2H,Ar-H]; 8.04[d,J=7.0Hz,2H,Ar-H]; 2.25[m,1H,cyclopropyl-H]; 0.80[m,4H,cyclopropyl-H]. | $C_{12}H_{11}N_3O_2$ 229.24 | 62.87 | 4.48 | 18.33 | |

TABLEAU I (suite)

| Ex | Ar | Methode | F°C | IR $cm^{-1}$ | $^1H$ RMN δ | Formule Pds. Mol. | Analyse % Calc | | | |
|---|---|---|---|---|---|---|---|---|---|---|
| | | | | | | | C | H | N | X |
| 8 | 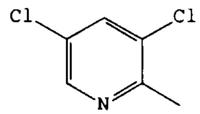 | B + C | 222-224 | 3080, 3005, 2190, 1630, 1525, 1430, 1405, 1220, 1105, 990, 885. | DMSO-$d_6$ 13.76(br s,1H,OH); 9.50-11.0 (br s,1H,NH); 8.62(d,J=1.6Hz,1H,Ar-H); 8.42(d,1H,Aryl-H); 2.22(m,1H,cyclo-propyl-H); 0.88(m,4H,cyclo-propyl-H). | $C_{12}H_9Cl_2N_3O_2$ 296.12 | 48.34<br>48.47 | 3.04<br>3.18 | 14.09<br>13.89 | 23.79<br>23.68 |

## ACTIVITES PHARMACOLOGIQUES

Procédés d'essai biochimique

Essai 1

Oedème de carrageenan des pattes de rat (PO-R)

Une heure suivant l'administration par voie orale des composés à l'essai ou du véhicule témoin à des groupes de rats (n = 6-12, mâle CFHB, intervalle de poids 160-180 g) 1 mg de carrageenan, dissous dans 0,2 ml de liquide physiologique, est injecté dans la patte arrière droite. Les pattes controlatérales reçoivent des injections de liquide physiologique témoin. Les réponses d'oedème des pattes sont contrôlées trois heures plus tard.

Essai 2

Hypersensibilité de type retardé d'oedème des pattes de souris (DTH-M)

Des groupes de souris (n = 8-10, mâle CD-1, intervalle de poids 25-30 g) sont sensibilisés par injection sous-cutanée de 1 mg de sérum albumine bovine méthylée (MBSA) dans des volumes de 0,2 ml de liquide physiologique/émulsion d'adjuvant complet de Freund (FCA). Les groupes témoins négatifs reçoivent des injections de liquide physiologique/émulsion FCA. Les réponses DTH d'oedème des pattes sont contrôlées vingt-quatre heures après l'épreuve de la patte arrière droite par 0,1 mg de MBSA dans des volumes de 0,05 ml de liquide physiologique au jour sept suivant la sensibilisation.
Les pattes controlatérales reçoivent des injections de liquide physiologique témoin. Les composés à l'essai et les véhicules témoins sont administrés par voie orale chaque jour aux jours quatre, cinq, six et deux fois au jour sept, une heure avant et six heures après l'épreuve par MBSA.

Essai 3

L'hypersensibilité de type retardé d'oedème des pattes de rat (DTH-R)

Des groupes de rats (n = 8-12, mâle CFHB, intervalle 160-180 g) sont sensibilisés par injection sous-cutanée à la base de la queue avec des volumes de 0,1 ml de FCA. Les groupes témoins négatifs reçoivent une injection d'adjuvant incomplet de Freund. Les réponses DTH d'oedème des pattes sont contrôlées vingt-quatre heures après l'épreuve des pattes arrières droites par 0,1 mg de MBSA dans par 0,4 mg d'antigène d'extrait de Mycobactérium tuberculosis dans 0,2 volume de liquide physiologique au jour sept après la sensibilisation. Les pattes controlatérales reçoivent des injections témoins de liquide physiologique.

Les composés à l'essai sont administrés par voie orale chaque jour aux jours quatre, cinq, six et deux fois au jour sept, une heure avant et six heures après l'épreuve antigénique.

Les résultats de ces essais sont présentés dans le tableau II. Les doses sont données en unités mg/kg p.o.

TABLEAU II

| Exemple | Essai 1 | | Essai 2 | | Essai 3 | |
|---|---|---|---|---|---|---|
| | % inhibition | Dose | % inhibition | Dose | % inhibition | Dose |
| 1 | 32 | 50 | Toxique<br>9 | 100<br>30 | 71<br>8 | 50<br>10 |
| 2 | 10 | 50 | 7 | 100 | 48 | 50 |
| 3 | 30 | 10 | 37 | 30 | 88<br>40 | 10<br>3 |
| 4 | 18 | 50 | 48 | 100 | 64 | 50 |
| 5 | 11 | 50 | Toxique<br>4 | 100<br>30 | Toxique<br>66 | 50<br>10 |
| 6 | -6 | 50 | 27 | 100 | 67 | 50 |
| 7 | -43 | 50 | 22 | 100 | 18 | 50 |
| 8 | -15 | 50 | 9 | 100 | 21 | 50 |

**Revendications**

**1.** 2-cyano-3-hydroxy-propenamides répondant à la formule générale (I) :

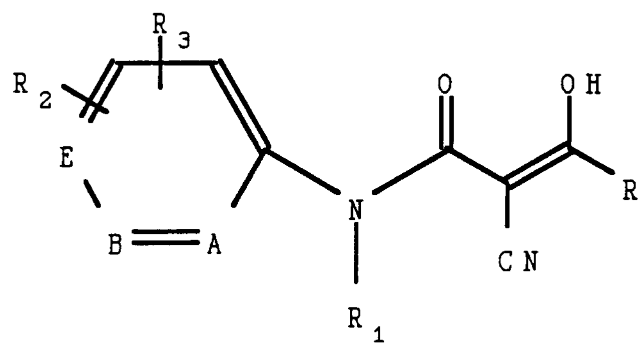

dans laquelle :

- A, B et E représentent chacun un groupement = CH - ou un atome d'azote, étant entendu que l'un au moins de A, B, ou E représente un atome d'azote,
- R représente un radical cycloalkyle renfermant de 3 à 6 atomes de carbone, un radical alkenyle renfermant de 2 à 6 atomes de carbone, un radical alkynyle renfermant de 2 à 6 atomes de carbone,
- $R_1$ représente un atome d'hydrogène, un radical alkyle renfermant de 1 à 3 atomes de carbone,
- $R_2$ et $R_3$ , identiques ou différents, représentent un atome d'hydrogène, un atome d'halogène, un groupement cyano, un groupement nitro, un radical alkyle, linéaire ou ramifié, renfermant de 1 à 6 atomes de carbone, un radical cycloalkyle renfermant de 3 à 6 atomes de carbone, un radical alcoxy, linéaire ou ramifié, renfermant de 1 à 6 atomes de carbone, un radical alkylthio renfermant de 1 à 6 atomes de carbone, un radical $-(CH_2)_m-CX_3$ , un radical $-O-(CH_2)_m-CX_3$ , un radical $-S-(CH_2)_m-CX_3$, $-O-(CX_2)_m-CX_3$, $-S-(CX_2)_m-CX_3$, radicaux dans lesquels m représente un nombre entier compris entre 0 et 3 et X représente un atome d'halogène, un groupement $-CO-R_4$ dans lequel $R_4$ représente un atome d'hydrogène, un radical alkyle, linéaire ou ramifié, renfermant de 1 à 6 atomes de carbone, un radical cycloalkyle renfermant de 3 à 6 atomes de carbone,

ou $R_2$ et $R_3$ , forment ensemble un groupement $-O-CH_2-O-$ , leurs formes tautomères, ainsi que leurs sels d'addition avec les bases minérales ou organiques.

**2.** 2-cyano-3-hydroxy-propenamides, tels que définis par la formule (I) de la revendication 1, caractérisés en ce que dans ladite formule (I), R représente un radical cyclopropyle, ou un des radicaux de formule :

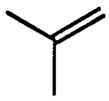
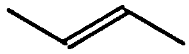
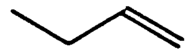
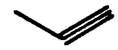

A, B, E, $R_1$, $R_2$ et $R_3$ ont la signification déjà indiquée, ainsi que leurs sels d'addition avec les bases minérales ou organiques.

**3.** 2-cyano-3-hydroxy-propenamides répondant à la formule (I) selon la revendication 1 ou 2, caractérisés en ce que dans ladite formule (I),
$R_1$ représente un atome d'hydrogène ou un radical méthyle, A, B, E, R, $R_2$ et $R_3$ ont la signification déjà indiquée, ainsi que leurs sels d'addition avec les bases minérales ou organiques.

**4.** 2-cyano-3-hydroxy-propenamides répondant à la formule (I) selon l'une quelconque des revendications 1, 2 ou 3, caractérisés en ce que dans ladite formule (I),
$R_1$ représente un atome d'hydrogène ou un radical méthyle, $R_2$ et $R_3$ , identiques ou différents, représentent un atome d'hydrogène, un atome de chlore ou de brome, un groupement cyano, un groupement nitro, un radical méthyle, un radical cyclopropyle, un radical méthoxy, un radical méthylthio, un radical $-(CH_2)_m-CF_3$ , un radical $-O-(CH_2)_m-CF_3$ , un radical $-S-(CH_2)_m-CF_3$, un radical $-O-(CF_2)_m-CF_3$ ou un radical $-S-(CF_2)_m-CF_3$, radicaux dans lesquels m représente un nombre entier compris entre 0 et 3, ou un groupement $-CO-R_4$ dans lequel $R_4$ représente un atome d'hydrogène, un radical méthyle, un radical cyclopropyle, ou $R_2$ et $R_3$, forment ensemble un groupement $-O-CH_2-O-$ ,
A, B, E et R ont la signification déjà indiquée, leurs formes tautomères, ainsi que leurs sels d'addition avec les bases minérales ou organiques.

**5.** 2-cyano-3-hydroxy-propenamides répondant à la formule (I) selon l'une quelconque des revendications 1, 2, 3 ou 4, caractérisés en ce que dans ladite formule (I),
R représente un radical cyclopropyle, $R_1$ représente un atome d'hydrogène ou un radical méthyle, $R_2$ et $R_3$ , identiques ou différents, représentent un atome d'hydrogène, un atome de chlore ou de brome, un groupement nitro, un radical méthyle, un radical trifluorométhyle, A, B, et E ont la signification déjà indiquée, leurs formes tautomères, ainsi que leurs sels d'addition avec les bases minérales ou organiques.

**6.** 2-cyano-3-hydroxy-propenamides répondant à la formule (I) selon l'une quelconque des revendications 1 à 5 dont les noms suivent :
- N-(2-chloropyrid-5-yl) 2-cyano-3-cyclopropyl-3-hydroxy-2-propenamide,
- 2-cyano-3-cyclopropyl-3-hydroxy-N-(4-methyl-5-nitropyrid-2-yl)-2-propenamide,
- 2-cyano-3-cyclopropyl-3-hydroxy-N-(5-trifluoromethylpyrid-2-yl)-2-propenamide,
- N-(5-chloropyrid-2-yl) 2-cyano-3-cyclopropyl-3-hydroxy-2-propenamide,
- N-(5-bromopyrid-2-yl) 2-cyano-3-cyclopropyl-3-hydroxy-2-propenamide,
- 2-cyano-3-cyclopropyl-3-hydroxy-N-(5-nitropyrid-2-yl)-2-propenamide,
- 2-cyano-3-cyclopropyl-3-hydroxy-N-(pyrid-4-yl)-2-propenamide,
- 2-cyano-3-cyclopropyl-3-hydroxy-N-(3,5-dichloropyrid-2-yl) 2-propénamide,

ainsi que leurs sels d'addition avec les bases minérales ou organiques.

**7.** Procédé de préparation des 2-cyano-3-hydroxypropenamides tels que définis par la formule (I) de la revendication 1, ainsi que de leurs sels, caractérisé en ce que l'on fait réagir un produit de formule (II) :

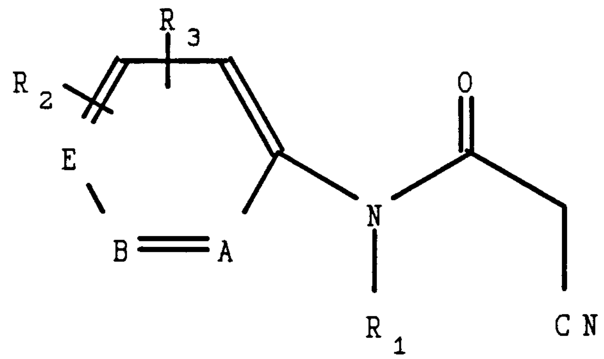

dans laquelle A, B, E, $R_1$, $R_2$ et $R_3$ ont la signification déjà indiquée, avec de l'hydrure de sodium, si nécessaire en présence d'un catalyseur, puis avec le dérivé fonctionnel d'un acide de formule (III):

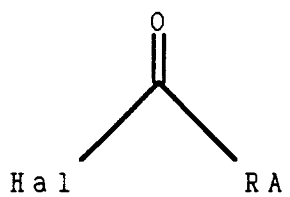

dans laquelle Hal représente un atome d'halogène et $R_A$ a la signification de R déjà indiquée, ou $R_A$ représente un radical R dûment protégé, pour obtenir un produit de formule ($I_A$):

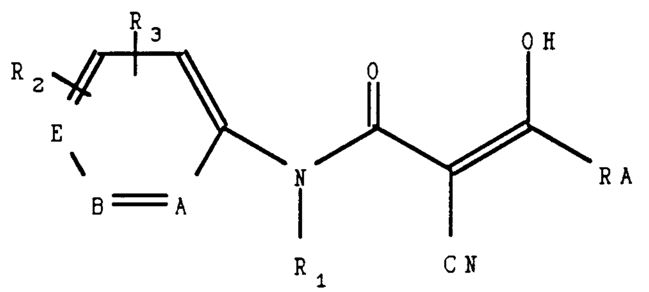

($I_A$)

dans laquelle A, B, E, $R_1$, $R_2$, $R_3$ et $R_A$ ont la signification déjà indiquée, puis, le cas échéant, déprotège le produit de formule ($I_A$) ainsi obtenu dans lequel $R_A$ représente un radical R dûment protégé, pour obtenir un produit de formule (I) correspondant, que l'on isole et si désiré salifie.

**8.** Procédé de préparation selon la revendication 7, caractérisé en ce que :

- la réaction du produit de formule (II) avec l'hydrure de sodium est effectuée au sein d'un solvant organique anhydre tel que le tétrahydrofuranne ou le dichloromethane en présence d'un catalyseur tel que l'imidazole,
- la réaction avec le dérivé formule (III) est effectuée de préférence au sein d'un solvant organique anhydre tel que le tétrahydrofuranne ou le dichlorométhane,
- la réaction avec le dérivé fonctionnel de l'acide de formule (III) est effectuée à basse température, ou à température ambiante.
- le dérivé fonctionnel de l'acide de formule (III) peut être par exemple le chlorure d'acide ou le fluorure d'acide,
- le radical R peut être protégé par un groupement arylseleno tel qu'un groupement phenylseleno,
- la déprotection peut être effectuée par oxydation au moyen d'un peroxyde tel que le peroxyde d'hydrogène en présence ou non d'un solvant ou d'un mélange de solvants organiques tel que le mélange methanol-dichloromethane.

**9.** Procédé de préparation des nouveaux 2-cyano-3-hydroxy-propenamides tels que définis par la formule (I) de la revendication 1, dans laquelle A, B, E, $R_1$, $R_2$ et $R_3$ ont la signification déjà indiquée, et R représente un radical cycloalkyle renfermant de 3 à 6 atomes de carbone, ainsi que de leurs sels, caractérisé en ce que l'on fait réagir un produit de formule (IV) :

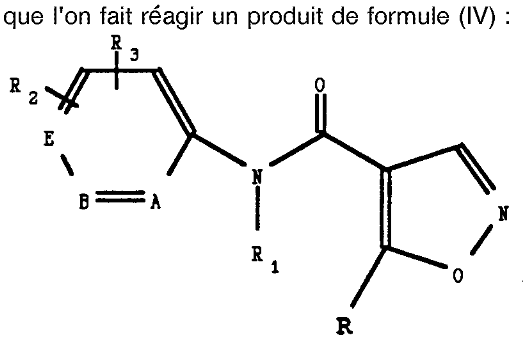

(IV)

dans laquelle A, B, E, R, $R_1$, $R_2$ et $R_3$ ont la signification déjà indiquée, avec une base forte pour obtenir le produit de formule (I) recherché, que l'on isole et si désiré salifie.

**10.** Médicaments, caractérisés en ce qu'ils sont constitués par les 2-cyano-3-hydroxy-propenamides tels que définis par la formule (I) de la revendication 1, ainsi que par leurs sels d'addition avec les bases pharmaceutiquement acceptables.

**11.** Médicaments, caractérisés en ce qu'ils sont constitués par les 2-cyano-3-hydroxy-propenamides tels que définis à l'une quelconque des revendications 2 à 6, ainsi que par leurs sels d'addition avec les bases pharmaceutiquement acceptables.

**12.** Compositions pharmaceutiques, caractérisées en ce qu'elles renferment à titre de principe actif, l'un au moins des médicaments tels que définis à l'une quelconque des revendications 10 ou 11.

## Claims

**1.** 2-cyano-3-hydroxy-propenamides corresponding to general formula (I):

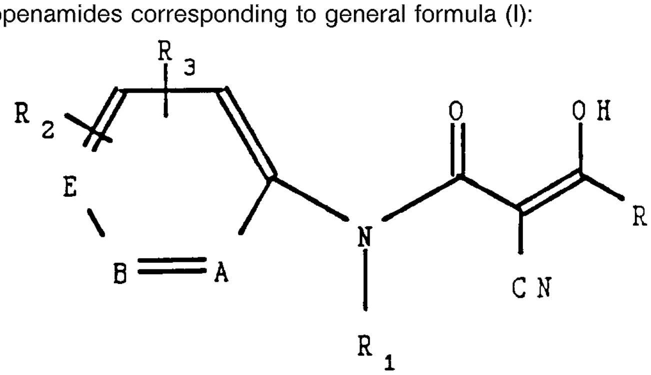

in which:

- A, B and E each represent a = CH - group or a nitrogen atom, it being understood that at least one of A, B or E represents a nitrogen atom,
- R represents a cycloalkyl radical containing 3 to 6 carbon atoms, an alkenyl radical containing 2 to 6 carbon atoms, an alkynyl radical containing 2 to 6 carbon atoms,
- $R_1$ represents a hydrogen atom, an alkyl radical containing 1 to 3 carbon atoms,
- $R_2$ and $R_3$, identical or different, represent a hydrogen atom, a halogen atom, a cyano group, a nitro group, a linear or branched alkyl radical containing 1 to 6 carbon atoms, a cycloalkyl radical containing 3 to 6 carbon atoms, a linear or branched alkoxy radical containing 1 to 6 carbon atoms, an alkylthio radical containing 1 to 6 carbon atoms, a $-(CH_2)_m-CX_3$ radical, an $-O-(CH_2)_m-CX_3$ radical, an $-S-(CH_2)_m-CX_3$, $-O-(CX_2)_m-CX_3$, $-S-(CX_2)_m-CX_3$ radical, in which radicals m

represents an integer comprised between 0 and 3 and X represents a halogen atom, a $-CO-R_4$ group in which $R_4$ represents a hydrogen atom, a linear or branched alkyl radical containing 1 to 6 carbon atoms, a cycloalkyl radical containing 3 to 6 carbon atoms,

or $R_2$ and $R_3$ form together an $-O-CH_2-O-$ group, their tautomeric forms, as well as their addition salts with mineral or organic bases.

**2.** 2-cyano-3-hydroxy-propenamides, as defined by formula (I) of claim 1, characterized in that in said formula (I), R represents a cyclopropyl radical, or one of the radicals of formula:

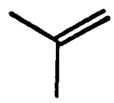
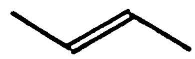
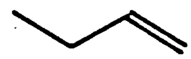
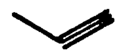

A, B, E, $R_1$, $R_2$ and $R_3$ have the meaning already indicated, as well as their addition salts with mineral or organic bases.

**3.** 2-cyano-3-hydroxy-propenamides corresponding to formula (I) according to claim 1 or 2, characterized in that in said formula (I),

$R_1$ represents a hydrogen atom or a methyl radical, A, B, E, R, $R_2$ and $R_3$ have the meaning already indicated, as well as their addition salts with mineral or organic bases.

**4.** 2-cyano-3-hydroxy-propenamides corresponding to formula (I) according to any one of claims 1, 2 or 3, characterized in that in said formula (I),

$R_1$ represents a hydrogen atom or a methyl radical, $R_2$ and $R_3$, identical or different, represent a hydrogen atom, a chlorine or bromine atom, a cyano group, a nitro group, a methyl radical, a cyclopropyl radical, a methoxy radical, a methylthio radical, a $-(CH_2)_m-CF_3$ radical, an $-O-(CH_2)_m-CF_3$ radical, an $-S-(CH_2)_m-CF_3$ radical, an $-O-(CF_2)_m-CF_3$ radical or an $-S-(CF_2)_m-CF_3$ radical, in which radicals m represents an integer comprised between 0 and 3, or a $-CO-R_4$ group in which $R_4$ represents a hydrogen atom, a methyl radical, a cyclopropyl radical, or $R_2$ and $R_3$ form together an $-O-CH_2-O-$group,

A, B, E and R have the meaning already indicated, their tautomeric forms, as well as their addition salts with mineral or organic bases.

**5.** 2-cyano-3-hydroxy-propenamides corresponding to formula (I) according to any one of claims 1, 2, 3 or 4, characterized in that in said formula (I),

R represents a cyclopropyl radical, $R_1$ represents a hydrogen atom or a methyl radical, $R_2$ and $R_3$, identical or different, represent a hydrogen atom, a chlorine or bromine atom, a nitro group, a methyl radical, a trifluoromethyl radical, A, B, and E have the meaning already indicated, their tautomeric forms, as well as their addition salts with mineral or organic bases.

**6.** 2-cyano-3-hydroxy-propenamides corresponding to formula (I) according to any one of claims 1 to 5 of which the names follow:

- N-(2-chloropyrid-5-yl) 2-cyano-3-cyclopropyl-3-hydroxy-2-propenamide,
- 2-cyano-3-cyclopropyl-3-hydroxy-N-(4-methyl-5-nitropyrid-2-yl)-2-propenamide,
- 2-cyano-3-cyclopropyl-3-hydroxy-N-(5-trifluoromethylpyrid-2-yl)-2-propenamide,
- N-(5-chloropyrid-2-yl) 2-cyano-3-cyclopropyl-3-hydroxy-2-propenamide,
- N-(5-bromopyrid-2-yl) 2-cyano-3-cyclopropyl-3-hydroxy-2-propenamide,
- 2-cyano-3-cyclopropyl-3-hydroxy-N-(5-nitropyrid-2-yl)-2-propenamide,
- 2-cyano-3-cyclopropyl-3-hydroxy-N-(pyrid-4-yl)-2-propenamide,
- 2-cyano-3-cyclopropyl-3-hydroxy-N-(3,5-dichloropyrid-2-yl) 2-propenamide,

as well as their addition salts with mineral or organic bases.

**7.** Preparation process for 2-cyano-3-hydroxy-propenamides as defined by formula (I) of claim 1, as well for their salts, characterized in that a product of formula (II):

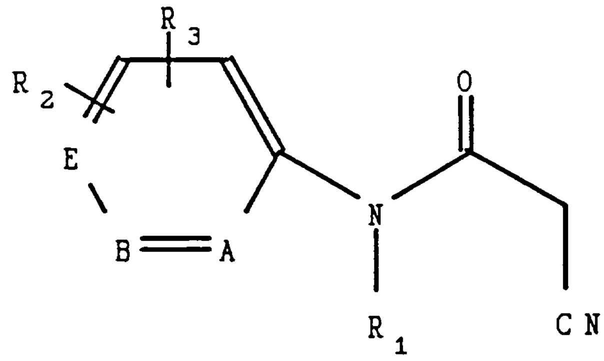

in which A, B, E, $R_1$, $R_2$ and $R_3$ have the meaning already indicated, is reacted with sodium hydride, if necessary in the presence of a catalyst, then with the functional derivative of an acid of formula (III):

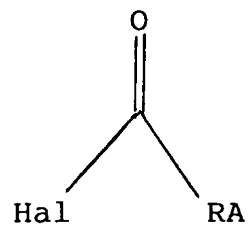

in which Hal represents a halogen atom and $R_A$ has the meaning of R already indicated, or $R_A$ represents a duly-protected radical R, in order to obtain a product of formula ($I_A$):

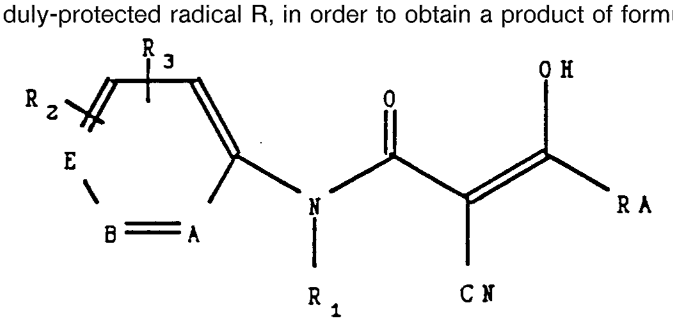

($I_A$)

in which A, B, E, $R_1$, $R_2$, $R_3$ and $R_A$ have the meaning already indicated, then, if appropriate, the product of formula ($I_A$) thus obtained in which $R_A$ represents a duly-protected radical R is deprotected, in order to obtain a corresponding product of formula (I), which is isolated and if desired salified.

**8.** Preparation process according to claim 7, characterized in that:
- the reaction of the product of formula (II) with sodium hydride is carried out in an anhydrous organic solvent such as tetrahydrofuran or dichloromethane in the presence of a catalyst such as imidazole,
- the reaction with the derivative of formula (III) is preferably carried out in an anhydrous organic solvent such as tetrahydrofuran or dichloromethane,
- the reaction with the functional derivative of the acid of formula (III) is carried out at low temperature, or at ambient temperature,
- the functional derivative of the acid of formula (III) can be for example the acid chloride or the acid fluoride,
- the radical R can be protected by an arylseleno group such as a phenylseleno group,
- the deprotection can be carried out by oxidation by means of a peroxide such as hydrogen peroxide in the presence or not of a solvent or a mixture of organic solvents such as a methanol-dichloromethane mixture.

9. Preparation process for new 2-cyano-3-hydroxy-propenamides as defined by formula (I) of claim 1, in which A, B, E, $R_1$, $R_2$ and $R_3$ have the meaning already indicated, and R represents a cycloalkyl radical containing 3 to 6 carbon atoms, as well as for their salts, characterized in that a product of formula (IV):

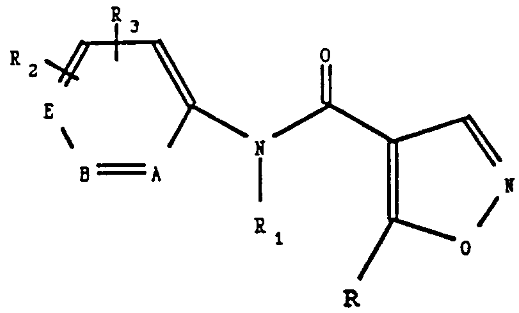

(IV)

in which A, B, E, R, $R_1$, $R_2$ and $R_3$ have the meaning already indicated, is reacted with a strong base in order to obtain the sought product of formula (I), which is isolated and if desired salified.

10. Medicaments, characterized in that they are constituted by the 2-cyano-3-hydroxy-propenamides as defined by formula (I) of claim 1, as well as by their addition salts with pharmaceutically acceptable bases.

11. Medicaments, characterized in that they are constituted by the 2-cyano-3-hydroxy-propenamides as defined in any one of claims 2 to 6, as well as by their addition salts with pharmaceutically acceptable bases.

12. Pharmaceutical compositions, characterized in that they contain as active ingredient, at least one of the medicaments as defined in any one of claims 10 or 11.

## Patentansprüche

1. 2-Cyano-3-hydroxypropenamide, die folgender allgemeiner Formel (I) entsprechen:

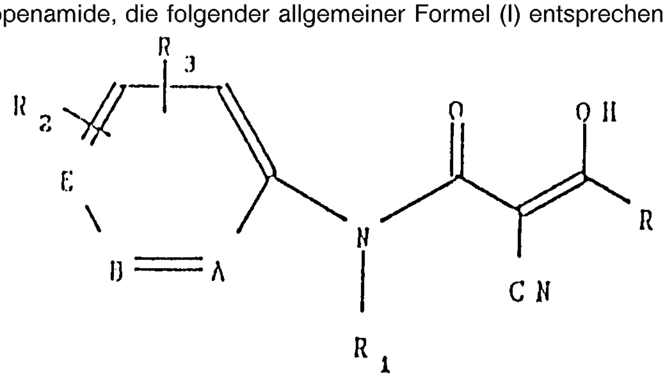

in der:
- A, B und E jeweils für eine =CH-Gruppierung oder ein Stickstoffatom stehen, wobei zumindest eines von A, B oder E für ein Stickstoffatom steht,
- R für einen Cycloalkylrest mit 3 bis 6 Kohlenstoffatomen, einen Alkenylrest mit 2 bis 6 Kohlenstoffatomen, einen Alkinylrest mit 2 bis 6 Kohlenstoffatomen steht,
- $R_1$ für ein Wasserstoffatom, einen Alkylrest mit 1 bis 3 Kohlenstoffatomen steht,
- $R_2$ und $R_3$, gleich oder verschieden, für ein Wasserstoffatom, ein Halogenatom, eine Cyanogruppe, eine Nitrogruppe, einen geradlinigen oder verzweigten Alkylrest mit 1 bis 6 Kohlenstoffatomen, einen Cycloalkylrest mit 3 bis 6 Kohlenstoffatomen, einen geradlinigen oder verzweigten Alkoxyrest mit 1 bis 6 Kohlenstoffatomen, einen Alkylthiorest mit 1 bis 6 Kohlenstoffatomen, einen $-(CH_2)_m-CX_3$-Rest, einen $-O-(CH_2)_m-CX_3$-Rest, einen $-S-(CH_2)_m-CX_3$-,$-O-(CX_2)_m-CX_3$-, $-S-(CX_2)_m-CX_3$-Rest stehen, Reste, in denen m eine ganze Zahl zwischen 0 und 3 darstellt und X für ein

Halogenatom, eine -CO-$R_4$-Gruppe steht, in der $R_4$ ein Wasserstoffatom, einen geradlinigen oder verzweigten Alkylrest mit 1 bis 6 Kohlenstoffatomen, einen Cycloalkylrest mit 3 bis 6 Kohlenstoffatomen darstellt,

oder $R_2$ und $R_3$ zusammen eine -O-$CH_2$-O-Gruppierung bilden,

ihre tautomeren Formen sowie ihre Additionssalze mit den Mineralbasen oder den organischen Basen.

**2.** 2-Cyano-3-hydroxypropenamide wie durch Formel (I) des Anspruchs 1 definiert, dadurch gekennzeichnet, daß in besagter Formel (I), R für einen Cyclopropylrest oder einen der Reste mit folgender Formel steht:

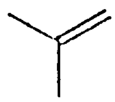
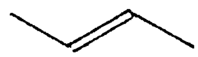
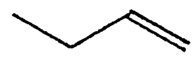
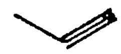

A, B, E, $R_1$, $R_2$ und $R_3$ die schon angegebene Bedeutung haben,
sowie ihre Additionssalze mit den Mineralbasen oder den organischen Basen.

**3.** 2-Cyano-3-hydroxypropenamide, die der Formel (I) nach Anspruch 1 oder 2 entsprechen, dadurch gekennzeichnet, daß in besagter Formel (I)
$R_1$ für ein Wasserstoffatom oder einen Methylrest steht,
A, B, E, R, $R_2$ und $R_3$ die schon angegebene Bedeutung haben,
sowie ihre Additionssalze mit den Mineralbasen oder den organischen Basen.

**4.** 2-Cyano-3-hydroxypropenamide, die der Formel (I) nach irgendeinem der Ansprüche 1, 2 oder 3 entsprechen, dadurch gekennzeichnet, daß in besagter Formel (I)
$R_1$ für ein Wasserstoffatom oder einen Methylrest steht,
$R_2$ und $R_3$, gleich oder verschieden, für folgendes stehen: ein Wasserstoffatom, ein Chlor- oder Bromatom, eine Cyanogruppe, eine Nitrogruppe, einen Methylrest, einen Cyclopropylrest, einen Methoxyrest, einen Methylthiorest, einen $-(CH_2)_m-CF_3$- Rest, einen $-O-(CH_2)_m-CF_3$-Rest, einen $-S-(CH_2)_m-CF_3$-Rest, einen $-O-(CF_2)_m-CF_3$-Rest oder einen $-S-(CF_2)_m-CF_3$-Rest, Reste, in denen m eine ganze Zahl zwischen 0 und 3 darstellt oder eine -CO-$R_4$-Gruppe, in der $R_4$ für ein Wasserstoffatom, einen Methylrest, einen Cyclopropylrest steht, oder $R_2$ und $R_3$ zusammen eine -O-$CH_2$-O-Gruppe bilden,
A, B, E und R die schon angegebene Bedeutung haben, ihre tautomeren Formen sowie ihre Additionssalze mit den Mineralbasen oder den organischen Basen.

**5.** 2-Cyano-3-hydroxypropenamide, die der Formel (I) nach irgendeinem der Ansprüche 1, 2, 3 oder 4 entsprechen, dadurch gekennzeichnet, daß in besagter Formel (I).
R für einen Cyclopropylrest steht, $R_1$ für ein Wasserstoffatom oder einen Methylrest steht, $R_2$ und $R_3$, gleich oder verschieden, für ein Wasserstoffatom, ein Chlor- oder Bromatom, eine Nitrogruppe, einen Methylrest, einen Trifluormethylrest stehen, A, B und E die schon angegebene Bedeutung haben, ihre tautomeren Formen sowie ihre Additionssalze mit den Mineralbasen oder den organischen Basen.

**6.** 2-Cyano-3-hydroxypropenamide, die der Formel (I) gemäß irgendeinem der Ansprüche 1 bis 5 entsprechen, mit folgenden Namen:

- N-(2-Chlorpyrid-5-yl)-2-cyano-3-cyclopropyl-3-hydroxy-2-propenamid,
- 2-Cyano-3-cyclopropyl-3-hydroxy-N-(4-methyl-5-nitropyrid-2-yl)-2-propenamid,
- 2-Cyano-3-cyclopropyl-3-hydroxy-N-(5-trifluormethylpyrid-2-yl)-2-propenamid,
- N-(5-Chlorpyrid-2-yl)-2-cyano-3-cyclopropyl-3-hydroxy-2-propenamid,
- N-(5-Brompyrid-2-yl)-2-cyano-3-cyclopropyl-3-hydroxy-2-propenamid
- 2-Cyano-3-cyclopropyl-3-hydroxy-N-(5-nitropyrid-2-yl)-2-propenamid,
- 2-Cyano-3-cyclopropyl-3-hydroxy-N-(pyrid-4-yl)-2-propenamid,
- 2-Cyano-3-cyclopropyl-3-hydroxy-N-(3,5-dichlorpyrid-2-yl)-2-propenamid sowie ihre Additionssalze mit den Mineralbasen oder den organischen Basen.

**7.** Verfahren zur Herstellung der 2-Cyano-3-hydroxypropenamide wie durch Formel (I) des Anspruchs 1 definiert sowie ihrer Salze, dadurch gekennzeichnet, daß man ein Produkt mit der Formel (II):

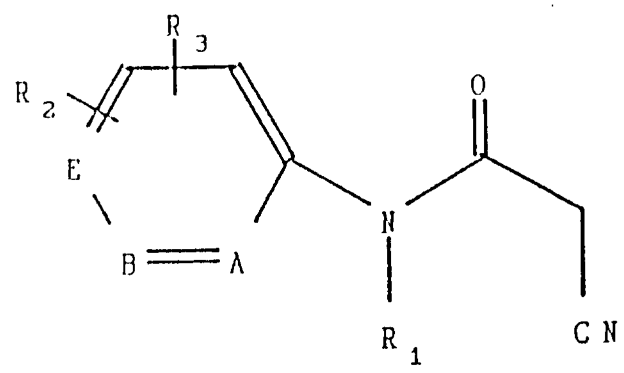

in der A, B, E, $R_1$, $R_2$ und $R_3$ die schon angegebene Bedeutung haben, mit Natriumhydrid reagieren läßt, wenn nötig in Gegenwart eines Katalysators, dann mit dem funktionellen Derivat einer Säure mit der Formel (III):

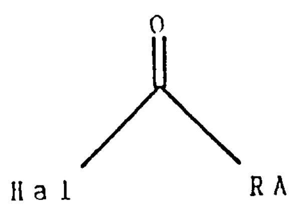

in der Hal für ein Halogenatom steht und $R_A$ die schon angegebene Bedeutung von R hat oder $R_A$ für einen gebührend geschützten Rest steht, um ein Produkt mit folgender Formel ($I_A$) zu erhalten:

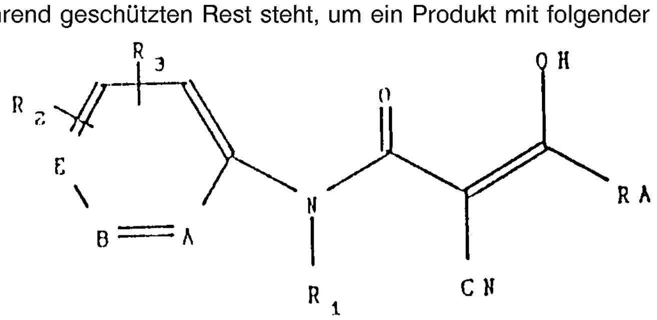

($I_A$)

in der A, B, E, $R_1$, $R_2$, $R_3$ und $R_A$ die schon angegebene Bedeutung haben, dann gegebenenfalls von dem so erhaltenen Produkt mit der Formel ($I_A$), in dem $R_A$ für einen gebührend geschützten Rest steht, die Schutzgruppe entfernt, um ein entsprechendes Produkt mit der Formel (I) zu erhalten, das man isoliert und, wenn gewüscht, in ein Salz überführt.

**8.** Herstellungsverfahren gemäß Anspruch 7, dadurch gekennzeichnet, daß
- die Reaktion des Produkts mit der Formel (II) mit dem Natriumhydrid in einem wasserfreien organischen Lösungsmittel wie Tetrahydrofuran oder Dichlormethan durchgeführt wird in Gegenwart eines Katalysators wie Imidazol,
- die Reaktion mit dem Derivat nach Formel (III) in Gegenwart eines wasserfreien organischen Lösungsmittels wie Tetrahydrofuran oder Dichlormethan durchgeführt wird,
- die Reaktion mit dem funktionellen Derivat der Säure mit der Formel (III) bei niedriger Temperatur oder bei Raumtemperatur durchgeführt wird,
- das funktionelle Derivat der Säure mit der Formel (III) zum Beispiel das Säurechlorid oder das Säurefluorid sein kann,
- der Rest R durch einen Arylselengruppe wie eine Phenylselengruppe geschützt sein kann,
- die Entfernung der Schutzgruppe durch Oxidation mit Hilfe eines Peroxids wie des Wasserstoffperoxids durchgeführt werden kann in Gegenwart oder Abwesenheit eines Lösungsmittels oder eines Gemischs von organischen Lösungsmitteln wie dem Methanol-Dichlormethangemisch.

**9.** Verfahren zur Herstellung der neuartigen 2-Cyano-3-hydroxypropenamide wie durch Formel (I) von Anspruch 1 definiert, in der A, B, E, $R_1$, $R_2$ und $R_3$ die schon angegebene Bedeutung haben und R für einen Cycloalkylrest mit 3 bis 6 Kohlenstoffatomen steht, sowie ihrer Salze, dadurch gekennzeichnet, daß man ein Produkt mit der Formel (IV):

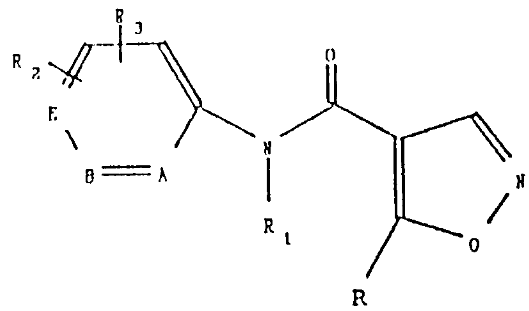

(IV)

in der A, B, E, R, $R_1$, $R_2$ und $R_3$ die schon angegebene Bedeutung haben, mit einer starken Base reagieren läßt, um das gesuchte Produkt mit der Formel (I) zu erhalten, das man isoliert und, wenn gewünscht, in ein Salz überführt.

**10.** Arzneistoffe, dadurch gekennzeichnet, daß sie aus den 2-Cyano-3-hydroxypropenamiden wie in Formel (I) von Anspruch 1 definiert bestehen sowie aus ihren Additionssalzen mit den pharmazeutisch verwendbaren Basen.

**11.** Arzneistoffe, dadurch gekennzeichnet, daß sie aus den 2-Cyano-3-hydroxypropenamiden wie in irgendeinem der Ansprüche 2 bis 6 definiert bestehen sowie aus ihren Additionssalzen mit den pharmazeutisch verwendbaren Basen.

**12.** Pharmazeutische Zusammensetzungen, dadurch gekennzeichnet, daß sie als Wirkstoff wenigstens einen der Arzneistoffe enthalten, wie sie in irgendeinem der Ansprüche 10 oder 11 definiert sind.